# EUROPEAN PATENT APPLICATION

(11) **EP 2 484 662 A1**
(43) Date of publication of application: **08.08.2012**
(21) Application number: 10820612.9
(22) Date of filing: 29.09.2010
(51) Int. Cl.: C07C 303/22, C07C 303/02, C07C 303/28, C07C 303/32, C07C 309/10, C07C 309/68, C07C 309/82, C09K 3/00, C25B 3/08

(54) **METHOD FOR PRODUCING PERFLUOROSULFONIC ACID HAVING ETHER STRUCTURE AND DERIVATIVE THEREOF, AND SURFACTANT CONTAINING FLUORINE-CONTAINING ETHER SULFONIC ACID COMPOUND AND DERIVATIVE THEREOF**

(30) Priority: 29.09.2009 JP 2009223975
(71) Applicant: Mitsubishi Materials Corporation, Chiyoda-ku Tokyo 100-8117 (JP); Mitsubishi Materials Electronic Chemicals Co., Ltd., Akita-shi Akita 010-8585 (JP)
(72) Inventor: KURUMAYA Mitsuo, Akita-shi Akita 010-8585 (JP); HONDA Tsunetoshi, Akita-shi Akita 010-8585 (JP); OMORI Kota, Akita-shi Akita 010-8585 (JP)
(74) Representative: Gille Hrabal
(86) International application number: PCT/JP2010/067002
(87) International publication number: WO 2011/040497

(57) **Abstract**

In this method, R_{H}²OR_{H}¹S0₂F is to hydrofluoric acid so as to form a thick solution (hydrogen bonded complex), and the solution is directly supplied to a liquid phase reaction system in which an F₂ gas is used. Alternatively, R_{H}²OR_{H}¹SO₂CL is added to hydrofluoric acid so as to be converted into R_{H}¹OR_{H}²SO₂F by discharging HCL, and the R_{H}¹OR_{H}²S0₂F is directly supplied to a liquid phase reaction system in which an F₂ gas is used. Consequently, fluorination can be carried out safely and a compound having an objective structure can be produced at low cost without isomerization or like.

## Description

### TECHNICAL FIELD

The present invention relates to a for producing a perfluorosulfonic acid having an ether structure (perfluoroalkoxy/perfluoroalkylsulfonic acid), a derivative thereof and a starting compound thereof, and to a surfactant containing a fluorine-containing ether sulfonic acid compound and a derivative thereof.
The present application claims priority on the basis of Japanese Patent Application No. 2009-223975, filed on September 29, 2009, the contents of which are incorporated herein by reference.

### BACKGROUND ART

Perfluorosulfonic acid and derivatives thereof (R_{F}SO₂X: wherein, R_{F} represents a group in which a hydrogen atom of the reacting hydrocarbon group is substituted with a fluorine atom, and X represents, for example, -OH or a halogen atom) have been used in surfactants, acid generators, ionic liquids, catalysts and the like. Among perfluorosulfonic acids, in particular, those having a perfluorooctane sulfonyl (C₈F₁₇SO₂-) structure having 8 carbon atoms are chemically stable; however, they have problems consisting of being resistant to degradation and accumulation in the body as a result thereof, and for this reason have begun to be restricted. In addition, similar restrictions have been enacted in the U.S. on perfluorosulfonic acids having 6 or more carbon atoms. Consequently, there is a need for an alternative compound that has less of an effect on the environment while maintaining the performance thereof.

One possible candidate for such an alternative compound is a compound obtained by introducing an ether structure into the aforementioned R_{F} group to obtain an R_{F}²OR_{F}¹- structure. Here, R_{F}¹ and R_{F}² represent groups in which hydrogen atoms of the each of the corresponding reacting hydrocarbon groups are substituted with fluorine atoms. A known example of a method used to produce these compounds consists of an R_{F}²OR_{F}¹SO₂X production method that uses perfluorovinylsulfonyl fluoride (CF₂=CFSO₂F) and perfluorohypofluorite (R_{F}OF) (Patent Document 1: Japanese Unexamined Patent Application, First Publication No. H6-128216). However, methods using perfluorovinylsulfonyl fluorite (CF₂=CFSO₂F) and perfluorohypofluorite (R_{F}OF) have problems consisting of using expensive fluorinated raw materials, requiring that the procedure be carried out at an extremely low temperature due to the low boiling point of the raw materials, being unable to arbitrarily select the ratio of the linear form (n-form) to isomer (i-form), and actually only obtaining a basic ethanesulfonyl structure.

With respect to other compounds, a method involving the synthesis of ether from alkoxide and alkyl halide (sulfonic acid) is commonly known as the Williamson method. However, in the case of producing a perfluoro compound by the Williamson method, the respective perfluorinated compounds are conventionally used as raw materials, thereby resulting in the same problems as those applicable to Patent Document 1.

### Prior Art Documents

### Patent Documents

Patent Document 1: Japanese Unexamined Patent Application, First Publication No. H6-128216

### DISCLOSURE OF THE INVENTION

### Problems to be Solved by the Invention

In order to solve the aforementioned problems, an object of the present invention is to provide a for producing a perfluorosulfonic acid having an ether structure (perfluoroalkoxy/ perfluoroalkylsulfonic acid) that enables a compound of a target structure to be produced inexpensively without causing isomerization and the like, a derivative thereof, and a starting compound thereof.
In addition, an object of the present invention is to provide a surfactant containing that derivative.

### Means for Solving the Problems

The inventors of the present invention found that a perfluorosulfonic acid having an ether structure other than an ethanesulfonyl structure (perfluoroalkoxy/perfluoroalkylsulfonic acid), and a derivative thereof, which were unable to be produced in the past, can be produced by forming a hydrocarbon compound having a desired carbon backbone and fluorinating that compound instead of forming a compound having an R_{F}²OR_{F}¹-structure using a fluorinated raw material.

In addition, the inventors of the present invention found that fluorination can be carried out safely and a compound of a target structure can be produced inexpensively without causing isomerization and the like by adding R_{H}²OR_{H}¹SO₂F to hydrofluoric acid to obtain a concentrated solution (hydrogen bonded complex), and either supplying this directly to a liquid
phase reaction system that uses F₂ gas, or adding hydrofluoric acid to R_{H}²OR_{H}¹SO₂Cl, discharging HCl to convert to R_{H}¹OR_{H}²SO₂F, and then supplying this to a liquid reaction system that uses F₂ gas.

On the basis of the aforementioned findings, the present invention provides a method for inexpensively producing a compound of a target structure without causing isomerization and the like for a perfluorosulfonic acid having an ether structure (perfluoroalkoxy/perfluoroalkylsulfonic acid) and a derivative thereof (R_{F}²OR_{F}¹SO₂X) . In addition, the present invention provides a method for producing starting compounds consisting of R_{H}²OR_{H}¹SO₂F and R_{H}²OR_{H}¹SO₂Cl. Moreover, the present invention provides a surfactant containing a derivative (R_{F}²OR_{F}¹SO₃M) .

According to the present invention, a method is provided for producing compounds having structures indicated below.
[1] A method for producing a fluorine-containing ether sulfonic acid compound comprising producing a perfluorosulfonic acid having an ether structure and a derivative R_{F}¹OR_{F}²SO₂X thereof (wherein, R_{F}¹ and R_{F}² represent groups in which hydrogen atoms in the R_{H}¹ and R_{H}² groups have been substituted with fluorine atoms and X represents -OH, an alkoxy group or a halogen) by perfluorinating a sulfonyl halide represented by the general formula R_{H}²OR_{H}¹SO₂Y (wherein, R_{H}¹ and
   R_{H}² respectively represent a hydrocarbon group having 1 to 4 carbon atoms and Y represents fluorine or chlorine).
[2] The method for producing a fluorine-containing ether sulfonic acid compound described in [1] above, wherein the sulfonyl halide is such that:
   the R_{H}¹ is a hydrocarbon group (linear or branched) having 3 carbon atoms in the case the R_{H}² is a hydrocarbon group having 1 carbon atom,
   the R_{H}¹ is a hydrocarbon group having 1 carbon atom, hydrocarbon group (linear or branched) having 3 carbon atoms or hydrocarbon group (linear or branched) having 4 carbon atoms in the case the R_{H}² is a hydrocarbon group (linear or branched) having 3 carbon atoms, and
   the R_{H}¹ is a hydrocarbon group having 1 carbon atom or a hydrocarbon group (linear or branched) having 3 carbon atoms in the case the R_{H}² is a hydrocarbon group (linear or branched) having 4 carbon atoms.
[3] The method for producing a fluorine-containing ether sulfonic acid compound described in [1] or [2] above, wherein the sulfonyl fluoride (R_{H}²OR_{H}¹SO₂F) described in [1] or [2] above is added to hydrofluoric acid to obtain a solution containing a hydrogen bonded complex, and this is supplied to a reaction solvent together with F₂ gas and then perfluorinated in a liquid phase to produce perfluorosulfonic acid having an ether structure and a derivative R_{F}¹OR_{F}²SO₂X thereof (wherein, R_{F}¹ and R_{F}² represent groups in which hydrogen atoms in the R_{H}¹ and R_{H}² groups have been substituted with fluorine atoms and X represents -OH, an alkoxy group or a halogen).
[4] The method for producing a fluorine-containing ether sulfonic acid compound described in [1] or [2] above, wherein the sulfonyl chloride (R_{H}²OR_{H}¹SO₂Cl) described in [1] or [2] above is added to hydrofluoric acid to convert to sulfonyl fluoride and obtain a solution containing a hydrogen bonded complex, and this is supplied to a reaction solvent together with F₂ gas and then perfluorinated in a liquid phase to produce a perfluorosulfonic acid having an ether structure and a derivative R_{F}¹OR_{F}²SO₂X thereof (wherein, R_{F}¹ and R_{F}² represent groups in which hydrogen atoms in the R_{H}¹ and R_{H}² groups have been substituted with fluorine atoms and X represents -OH, an alkoxy group or a halogen).
[5] The method for producing a fluorine-containing ether sulfonic acid compound described in [1] or [2] above, wherein the perfluorination is carried out by electrolytically fluorinating the sulfonyl fluoride (R_{H}²OR_{H}¹SO₂F) described in [1] or [2] above in anhydrous hydrofluoric acid.
[6] A production method of the perfluorosulfonic acid having an ether structure and a derivative R_{F}¹OR_{F}²SO₂X thereof described in [3] or [4] above, comprising carrying out the reaction by preliminarily adding and suspending NaF or KF in the liquid phase fluorination reaction liquid as an adsorbent of hydrofluoric acid.
[7] A production method of the perfluorosulfonic acid having an ether structure and a derivative R_{F}¹OR_{F}²SO₂X thereof described in any of [1] to [6] above, comprising converting a fluorination reaction product (R_{F}²OR_{F}¹SO₂F) in the reaction liquid to a sulfonic acid ester (R_{F}²OR_{F}¹SO₂OR³) using a base and an alcohol R³OH, and then separating and purifying by distillation.

In addition, the present invention provides a method for producing a hydrocarbon sulfonyl fluoride having an ether structure (alkoxyalkylsulfonyl fluoride) composed in the manner described below that is useful as a starting compound of the production methods described in [1] to [7] above.
[8] A method for producing a hydrocarbon sulfonyl fluoride having an ether structure (alkoxyalkylsulfonyl fluoride), comprising a step wherein an alkoxide, obtained by reacting CH₃OM, C₂H₅OM or a linear or branched alcohol having 3 to 4 carbon atoms with a metal M, M-H or CH₃OM (wherein, M represents Na, K or Li), is reacted with X¹-R_{H}¹-SO₂-X² (wherein, X¹ represents Cl or Br, R_{H}¹ represents a linear alkyl group having 1 to 4 carbon atoms, and X² represents ONa, OK, Cl or Br) to synthesize R²-O-R_{H}¹-SO²-X² (wherein, R²-O- represents an alkoxy group equivalent to the alkoxide), followed by subjecting to the action of a chlorinating agent to obtain R_{H}²-O-R_{H}¹-SO₂-Cl, and further converting to R_{H}²-O-R_{H}¹-SO₂-F in an aqueous solution containing KF.
[9] A method for producing a hydrocarbon sulfonyl fluoride having an ether structure (alkoxyalkylsulfonyl fluoride), comprising a step wherein CH₃OH, C₂H₅OH or a linear or branched alcohol having 3 to 4 carbon atoms is reacted directly with 1,3-propane sultone or 1,4-butane sultone to synthesize R²-O-R_{H}¹-SO₂-OH (wherein, R²-O- represents an alkoxy group equivalent to the alkoxide, and R_{H}¹ represents a linear alkylene derived from the sultone) , followed by subjecting to the action of a chlorinating agent to obtain R_{H}²-O-R_{H}¹-SO₂-Cl, and further converting to R_{H}²-O-R_{H}¹-SO₂-F in a KF-organic solvent-water system.
[10]A method for producing a hydrocarbon sulfonyl fluoride having an ether structure (alkoxyalkylsulfonyl fluoride), comprising a step wherein an alkoxide, obtained by reacting CH₃OM, C₂H₅OM or a linear or branched alcohol having 3 to 4 carbon atoms with a metal M, M-H or CH₃OM (wherein, M represents Na, K or Li), is reacted directly with 1,3-propane sultone or 1,4-butane sultone to synthesize R²-O-R_{H}¹-SO₂*-*OM (wherein, R²-O-represents an alkoxy group equivalent to the alkoxide, and R_{H}¹ represents a linear alkylene derived from the sultone), followed by subjecting to the action of a chlorinating agent to obtain R_{H}²-O-R_{H}¹-SO₂-Cl, and further converting to R_{H}²-O-R_{H}¹-SO₂-F in an aqueous solution containing KF.

[11] A fluorine-containing ether sulfonic acid compound that is a compound represented by general formula R_{F}¹-OR_{F}²SO₂X (wherein, R_{F}¹ and R_{F}² respectively represent a perfluoroalkyl group having 1 to 4 carbon atoms, and X represents -OH, an alkoxy or a halogen) , wherein
   the R_{F}¹ is a perfluoroalkyl group (linear or branched) having 3 carbon atoms in the case the R_{F}² is a perfluoroalkyl group having 1 carbon atom,
   the R_{F}¹ is a perfluoroalkyl group having 1 carbon atom, a perfluoroalkyl group (linear or branched) having 3 carbon atoms or a perfluoroalkyl group (linear or branched) having 4 carbon atoms in the case the R_{F}² is a perfluoroalkyl group (linear) having 3 carbon atoms, and
   the R_{F}¹ is a perfluoroalkyl group having 1 carbon atom or a perfluoroalkyl group (linear or branched) having 3 carbon atoms in the case the R_{F}² is a perfluoroalkyl group (linear) having 4 carbon atoms.
[12]A surfactant that contains a compound represented by general formula R_{F}¹OR_{F}²S0₃M (wherein, R_{F}¹ and R_{F}² respectively represent a perfluoroalkyl group having 1 to 4 carbon atoms, and M represents Li, Na, K or NH₄), wherein
   the R_{F}¹ is a perfluoroalkyl group (linear or branched) having 3 carbon atoms in the case the R_{F}² is a perfluoroalkyl group having 1 carbon atom,
   the R_{F}¹ is a perfluoroalkyl group having 1 carbon atom, a perfluoroalkyl group (linear or branched) having 3 carbon atoms or a perfluoroalkyl group (linear or branched) having 4 carbon atoms in the case the R_{F}² is a perfluoroalkyl group (linear) having 3 carbon atoms, and
   the R_{F}¹ is a perfluoroalkyl group having 1 carbon atom or a perfluoroalkyl group (linear or branched) having 3 carbon atoms in the case the R_{F}² is a perfluoroalkyl group (linear) having 4 carbon atoms.

### Effects of the invention

According to the present invention, molecular design can be carried out with a comparatively inexpensive hydrocarbon compound,and a perfluoro compound can be obtained while maintaining the structure thereof. In addition, not only is the cost low, but the yield is favorable. Consequently, the present invention is highly useful as a method for synthesizing a diverse range of novel compounds for use as alternative compounds to conventional perfluoroalkylsulfonic acids and derivatives thereof.

In addition, the present invention is also able to provide a surfactant containing a novel compound.

### BEST MEANS FOR CARRYING OUT THE INVENTION

The following provides a detailed explanation of the present invention.

### [Perfluorination]

### (First Aspect)

In a basic aspect of the present invention, a sulfonyl fluoride R_{H}²OR_{H}¹SO₂F (wherein, R_{H}¹ and R_{H}² respectively represent a hydrocarbon group having 1 to 4 carbon atoms) is added to hydrofluoric acid to obtain a solution containing a hydrogen bonded complex. F₂ gas is supplied to a reaction solvent that is stable with respect to F₂ gas and this is supplied to the aforementioned solution followed by carrying out perfluorination in a liquid phase. Furthermore, although perfluorination can also actually be carried out by a method consisting of reacting directly with F₂ gas without using a solvent or by a solid phase reaction using CoF₃, since it is difficult to control the reaction and there is typically the problem of low yield attributable to decomposition and the like, perfluorination is advantageously carried out in the liquid phase.

Here, hydrofluoric acid may be anhydrous hydrofluoric acid or may contain water up to abort 10% by weight. The amount of the hydrofluoric acid is preferably 0.5 to 10 times the number of moles of the raw material and particularly preferably 1 to 3 times the number of moles of the raw material.

Examples of reaction solvents that are stable with respect to F₂ gas include perfluoroalkanes, perfluoroethers, perfluoropolyethers and perfluorotrialkylamines that can be acquired as industrial products or reagents, and these can be used alone or as a mixture thereof. Although chlorofluorocarbons can also be used, these have a considerable effect on the environment in comparison with the aforementioned solvents, thereby making them undesirable. The amount of the reaction solvent is preferably 0.5 mol/L to 0.01 mol/L and more preferably 0.2 mol/L to 0.05 mol/L based on the raw material.

In addition, a compound capable of being fluorinated may also be present for the purpose of regulating the reaction. Compounds having a double bond between carbon atoms such as benzene or hexafluorobenzene can be used. The amount thereof is preferably 1 to 50 mol% based on the raw material, and may be added to the starting solution or may be supplied to the reaction liquid after separately dissolving in the reaction solvent. In addition, ultraviolet light may also be radiated for the same purpose.

The F₂gas may be diluted with an inert gas. Examples of such inert gases that can be used include nitrogen gas, helium gas and argon gas. Among these, nitrogen gas is economically preferable. The concentration of F₂ in the gas is determined so that the reaction proceeds suitably, and may be changed corresponding to the progression of the reaction. The concentration of the F₂ gas is preferably 1 to 50 vol% and more preferably 10 to 30 vol%. The reaction temperature is preferably from -80°C to equal to or lower than the boiling point of the solvent and more preferably -30°C to 30°C from the viewpoint of control.

### [Second Aspect]

Perfluorination may be carried out by adding a sulfonyl chloride R_{H}²OR_{H}¹SO₂Cl to hydrofluoric acid to convert to R_{H}¹OR_{H}²SO₂F and obtain a solution containing a hydrogen bonded complex in a second aspect instead of the aforementioned first aspect. Perfluorination can be carried out safely by converting the sulfonyl chloride R_{H}²OR_{H}¹SO₂Cl to sulfonyl fluoride R_{H}¹OR_{H}²SO₂F by discharging HCl in a reaction with hydrofluoric acid, or by supplying directly to a a liquid phase reaction using F₂ gas.

The hydrofluoric acid produced as a by-product in the liquid phase reaction that user F₂ and the hydrofluoric acid added to the starting solution are preferably promptly removed. A column packed with NaF pellets may be attached to the exhaust gas line of the reaction apparatus to adsorb hydrofluoric acid, and a condenser may be provided in the backflow thereof followed by returning the reaction liquid to the reactor. In addition, the reaction is more preferably carried out by preliminarily adding NaF or KF to a liquid phase fluorination reaction liquid and suspending therein. Yield can be improved by adding and suspending NaF and the like. The NaF and the like can be used in any of the forms of powder, pellets or crystals. The amount of NaF and the like added is preferably 0.5 to 10 times the number of moles of the hydrofluoric acid produced as a by-product in the reaction and the hydrofluoric acid added to the starting solution, and particularly preferably 1 to 3 times that number of moles. If the added amount is excessively low, progression of the reaction is inhibited, and it becomes necessary to provide a separately step for removing the excess hydrofluoric acid. If the amount added is excessively high, the process becomes uneconomical and the burden on filtration or other equipment or apparatuses increases.

The fluorination reaction product (R_{F}²OR_{F}¹SO₂F) present in the reaction liquid obtained in this manner may be further converted to a sulfonic acid ester (RF²OR_{F}¹SO₂OR³) using a base (carbonate of alkaline metal or organic base such as triethylamine) and an alcohol R³OH. Separation and purification by distillation can be carried out easily as a result of converting to this ester compound. In addition, the fluorination reaction product may also be isolated as R_{F}²OR_{F}¹SO₃M by allowing MOH (where, M represents an alkaline metal) to act on a perfluorosulfonic acid ester (R_{F}²OR_{F}¹SO₂OR³), or may be isolated as R_{F}²OR_{F}¹SO₃H by treating with a mineral acid (such as H₂SO₄ or HCl).

Alternatively, the fluorination reaction product (R_{F}²OR_{F}¹SO₂F) may be isolated as R_{F}²OR_{F}¹SO₃M by allowing MOH (where, M represents an alkaline metal) or may be isolated as R_{F}²OR_{F}¹SO₃H by treating with a mineral acid (such as H₂SO₄ or HCl) at the stage the fluorination reaction product is present in the reaction liquid.

### (Third Aspect)

Perfluorination may also be carried out by electrolytically fluorinating a sulfonyl fluoride R_{H}²OR_{H}¹SO₂F in anhydrous hydrofluoric acid as a third aspect instead of the aforementioned first aspect and second aspect. Here, the sulfonyl fluoride used as an electrolysis raw material can be easily produced by fluorine replacement by adding potassium fluoride (KF) and the like to sulfonyl chloride R_{H}²OR_{H}¹SO₂Cl.

Electrolytic fluorination specifically consists of using sulfonyl fluoride R_{H}²OR_{H}¹SO₂F as a raw material, introducing this into an electrolytic bath along with hydrofluoric acid, and then electrolyzing under normal pressure in a nitrogen gas atmosphere. As a result, the hydrocarbon groups R_{H}¹ and R_{H}² of the sulfonyl fluoride are replaced with fluorine resulting in the formation of a fluorination reaction product (R_{F}²OR_{F}¹SO₂F).

As has been described above, according to the present invention, any of perfluorosulfonic acid having an ether structure and a derivative R_{F}¹OR_{F}²SO₂X thereof (wherein, R_{F}¹ and R_{F}² represent groups in which hydrogen atoms in the aforementioned R_{H}¹ and R_{H}² groups are substituted with fluorine, and X represents -OH, an alkoxy group or a halogen) can be produced.

The following lists specific examples of compounds produced according to the production method of the present invention (and X in the compounds is the same as defined above). All of the compounds listed here are thought to be novel compounds.
CF₃O(CF₂)₃SO₂X, n-C₃F₇O(CF₂)₃SO₂X, CF₃O(CF₂)₄SO₂X, CF₃OCF₂SO₂X, n-C₃F₇OCF₂SO₂X, CF₃CF(CF₃)OCF₂SO₂X, n-C₄F₉OCF₂SO₂X, C₂F₅CF(CF₃)OCF₂SO₂X, (CF₃)₃COCF₂SO₂X, n-C₃F₇O(CF₂)₂SO₂X, CF₃CF(CF₃)O(CF₂)₃SO₂X, n-C₄F₉(CF₂)₃SO₂X, C₂F₅CF(CF₃)O(CF₂)₃SO₂X, (CF₃)₃CO(CF₂)₃SO₂X, n-C₃F₇O(CF₂)₄SO₂X, CF₃CF(CF₃)O(CF₂)₄SO₂X

### [Surfactant]

A compound (salt of perfluorosulfonic acid) that is a derivative of perfluorosulfonic acid represented by the general formula R_{F}¹OR_{F}²SO₃M (wherein, R_{F}¹ and R_{F}² respectively represent a perfluoroalkyl group having 1 to 4 carbon atoms, and M represents Li, Na, K, or NH₄) is formed by hydrolyzing the aforementioned derivative R_{F}¹OR_{F}²SO₂X (wherein, R_{F}¹ and R_{F}² represent groups in which hydrogen atoms in the aforementioned R_{H}¹ and R_{H}² groups are substituted with fluorine atoms, and X represents -OH, an alkoxy group or a halogen) with an aqueous alkaline solution.

Here, the basic carbon backbone of the aforementioned derivative R_{F}¹OR_{F}²SO₂X is such that:
R_{F}¹ is preferably a perfluoroalkyl group (linear or branched) in the case R_{F}² is a perfluoroalkyl group having 1 carbon atom,
R_{F}¹ is preferably a perfluoroalkyl group having 1 carbon atom, a perfluoroalkyl group (linear or branched) having 3 carbon atoms or a perfluoroalkyl group (linear or branched) having 4 carbon atoms in the case R_{F}² is a perfluoroalkyl group (linear) having 3 carbon atoms, and
R_{F}¹ is preferably a perfluoroalkyl group having 1 carbon atom or a perfluoroalkyl group (linear or branched) having 3 carbon atoms in the case R_{F}² is a perfluoroalkyl group (linear) having 4 carbon atoms.

In addition, examples of the aqueous alkaline solution that can be used include aqueous lithium hydroxide (LiOH), aqueous sodium hydroxide (NaOH), aqueous potassium hydroxide (KOH) and aqueous ammonia (NH₃).

An aqueous solution of a salt of perfluorosulfonic acid represented by the general formula R_{F}¹OR_{F}²SO₃M (wherein, R_{F}¹ and R_{F}² respectively represent a perfluoroalkyl group having 1 to 4 carbon atoms, and M represents Li, Na, K, or NH₄) can be used as a surfactant with respect to water.

### [Starting Compound]

Although an alkylsulfonic acid derivative having an ether structure serving as the starting compound of the perfluorosulfonic acid derivative having an ether structure according to the present invention as previously described can be produced by various methods, an aspect of the present invention provides the production methods described below. Furthermore, an example of a chlorinating agent used in the following production methods is SOCl₂.

A first production method is as described below.
A method for producing a hydrocarbon sulfonyl fluoride having an ether structure (alkoxyalkylsulfonyl fluoride), comprising a step wherein an alkoxide, obtained by reacting CH₃OM, C₂H₅OM or a linear or branched alcohol having 3 to 4 carbon atoms and a metal M, M-H or CH₃OM (wherein, M represents Na, K or Li), is reacted with X¹-R_{H}¹-SO₂-X² (wherein, X¹ represents Cl or Br, R_{H}¹ represents a linear alkyl group having 1 tao 4 carbon atoms, and X² represents ONa, OK, Cl or Br) to synthesize R²-O-R_{H}¹-SO₂-X² (wherein, R²-O- represents an alkoxy group equivalent to the alkoxide), followed by subjecting to the action of a chlorinating agent to obtain R_{H}²-O-R_{H}¹-SO₂-Cl, and further converting to R_{H}²-O-R_{H}¹-SO₂-F in an aqueous solution containing KF.

A second production method is as described below.
A method for producing a hydrocarbon sulfonyl fluoride having an ether structure (alkoxyalkylsulfonyl fluoride), comprising a step wherein a CH₃OH, C₂H₅OH or a linear or branched alcohol having 3 to 4 carbon atoms is reacted directly with 1,3-propane sultone or 1,4-butane sultone to synthesize R²-O-R_{H}¹-SO₂-OH (wherein, R²-O- represents an alkoxy group equivalent to the alkoxide, and R_{H}¹ represents a linear alkylene derived from the sultone), followed by subjecting to the action of a chlorinating agent to obtain R_{H}²-O-R_{H}¹-SO₂-Cl, and further converting to R_{H}²-O-R_{H}¹-SO₂-F in a KF-organic solvent-water system. An acid catalyst such as CF₃SO₃H may be added during the reaction between the alcohol and sultone.

A third production method is as described below.
A method for producing a hydrocarbon sulfonyl fluoride having an ether structure (alkoxyalkylsulfonyl fluoride), comprising a step wherein an alkoxide, obtained by reacting CH₃OM, C₂H₅OM or a linear or branched alcohol having 3 to 4 carbon atoms and a metal M, M-H or CH₃OM (wherein, M represents Na, K or Li), is reacted directly with 1,3-propane sultone or 1,4-butane sultone to synthesize R²-O-R_{H}¹-SO₂-OM (wherein, R²-O-represents an alkoxy group equivalent to the alkoxide, and R_{H}¹ represents a linear alkylene derived from the sultone), followed by subjecting to the action of a chlorinating agent to obtain R_{H}²-O-R_{H}¹-SO₂-Cl, and further converting to R_{H}²-O-R_{H}¹-SO₂-F in an aqueous solution containing KF.

The following lists specific examples of the R_{H}² OR_{H}¹SO₂X produced according to the methods described above. In the following chemical formulas, X is the same as previously defined, and is a halogen, for example. Examples of halogens include F and Cl.
CH₃OCH₂SO₂X, n-C₃H₇OCH₂SO₂X, CH₃CH(CH₃)OCH₂SO₂X, n-C₄H₉OCH₂SO₂X, C₂H₅CH(CH₃)OCH₂SO₂X, (CH₃)₃COCH₂SO₂X, n-C₃H₇O(CH₂)₂SO₂X, CH₃CH(CH₃)O(CH₂)₂SO₂X, C₂H₅CH(CH₃)O(CH₂)₂SO₂X, (CH₃)₃CO(CH₂)₂SO₂X, CH₃CH(CH₃)O(CH₂)₃SO₂X, C₂H₅CH(CH₃)O(CH₂)₃SO₂X, (CH₃)₃CO(CH₂)₃SO₂X, n-C₃H₇O(CH₂)₄SO₂X, CH₃CH(CH₃)O(CH₂)₄SO₂X, C₂H₅CH(CH₃)O(CH₂)₄SO₂X, (CH₃)₃CO(CH₂)₄SO₂X, and so on.

### EXAMPLES

The following provides a detailed description of the present invention using examples and reference examples. Furthermore, the present invention is not limited to these examples. In the following examples, identification and confirmation of the products were carried out by GC-MS (EI, 70 eV) and ¹H-NMR (270 MHz, TMS standard) /¹⁹F-NMR (254 MHz, CCl₃F standard) unless specifically stated otherwise. A Teflon® vessel made of PFA was used for the reaction vessel.

### Example 1-1-1 [Production of CH₃O(CH₂)₃SO₂F]

### [Synthesis example of alkylsulfonic acid derivative having ether structure (starting compound of perfluorosulfonic acid derivative having ether structure according to present invention) using first production method]

11.25 g (50 mmol) of sodium 3-bromopropanesulfonate and 50 ml of methanol were charged into a 200 ml glass four-mouth flask equipped with a reflux condenser, thermometer and stirrer followed by heating and refluxing in an oil bath. 75 g (75 mmol) of 28% sodium methylate were dropped therein over the course of 1 hour and further allowed to react for 22 hours while continuing to reflux. After allowing the reactants to cool, water was added until the liquid became transparent followed by neutralizing in 1:1 dilute hydrochloric acid, transferring to a recovery flask, and concentrating and drying with a rotary evaporator. 60 g of chloroform and 0.3 g of N,N-dimethylformamide (DMF) as catalyst were added to the dried product, a forked connecting pipe was attached, and 23.8 g (200 mmol) of thionyl chloride were dropped in at room temperature followed by allowing to react for 17 hours while heating and refluxing. in an oil bath. After concentrating the reaction liquid under reduced pressure, a solution containing 50 g of chloroform and 4.35 g (75 mmol) of potassium fluoride dissolved in 30 g of water was added, followed by stirring for 24 hours at room temperature. The reaction liquid was separated, the chloroform layer was washed 3 times with water, dried with anhydrous magnesium sulfate and concentrated with a rotary evaporator to obtain the target compound by vacuum distillation using a packed column. The amount obtained was 4.81 g, GC purity was 99%, yield was 61% and the boiling point was 87°C to 89°C at 2.67 kPa. ¹H-NMR (solvent: CDCl₃, ppm): 2.18 (m,2H), 3.35 (s,3H), 3.51 (m,4H). ¹⁹F-NMR (solvent: CDCl₃, ppm): 52.73 (t,1F).

### [Example 1-1-1]

Synthesis of sodium 3-methoxy-1-propanesulfonate and 3-methoxy-1-propanesulfonyl chloride/flouride from sodium 3-bromo-1-propanesulfonate and sodium methylate BrCH₂CH₂CH₂SO₃Na + CH₃ONa → CH₃OCH₂CH₂CH₂SO₃Na CH₃OCH₂CH₂CH₂SO₃Na + SOCl₂ → CH₃OCH₂CH₂CH₂SO₂Cl CH₃OCH₂CH₂CH₂SO₂Cl + KF → CH₃OCH₂CH₂CH₂SO₂F

### example 1-1-2 [Production of CH₃O(CH₂)₃SO₂F]

### [Synthesis example of alkylsulfonic acid derivative having ether structure (starting compound of perfluorosulfonic acid derivative having ether structure according to present invention) using second production method]

25.6 g (0.8 mol) of methanol and 24.4 g (0.2 mol) of 1,3-propane sultone were charged into the same apparatus as that of Example 1-1-1 and allowed to for 3 white refluxing. The reactants were transferred to a recovery flask and concentrated with a rotary evaporator to obtain a viscous liquid. 150 g of chloroform and 1 g of N,N-dimethylformamide (DMF) as catalyst were added thereto, a forked connecting tube was attached and 95.2 g (0.8 mol) of thionyl chloride were dropped in at room temperature, followed by reacting for 17 hours while heating and refluxing in an oil bath. After concentrating the reaction liquid under reduced pressure, a solution containing 150 g of chloroform and 17.4 g (0.3 mol) of potassium fluoride dissolved in 80 g of water was added followed by stirring for 24 hours at room temperature. The reaction liquid was separated, the chloroform layer was washed 3 times with water, dried with anhydrous magnesium sulfate and concentrated with a rotary evaporator to obtain the target compound by vacuum distillation using a packed column. The amount obtained was 13.41 g, GC purity was 98.5%, and yield was 78%.

### [Example 1-1-2]

### Synthesis of 3-methoxy-1-propanesulfonate and 3-methoxy-1-propanesulfonyl chloride/flouride from 1,3-propane sultone and methanol

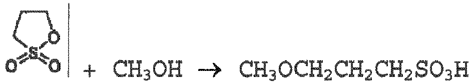

CH₃OCH₂CH₂CH₂SO₃H + SOCl₂ → CH₃OCH₂CH₂CH₂SO₂Cl

CH₃OCH₂CH₂CH₂SO₂Cl + KF → CH₃OCH₂CH₂CH₂SO₂F

### example 1-2-1 [production of CF₃O (CF₂) ₃SO₂F and CF₃O (CF₂) ₃SO₂OCH₂CF₃]

### [a] Production of CF₃O (CF₂) ₃SO₂F

0.44 g (22 mmol) of anhydrous hydrofluoric acid were placed in a fluorine resin PFA container having a volume of 7 ml in an ice bath, followed by slowly dropping in 1.56 g (10 mmol) of the starting compound CH₃O (CH₂) ₃SO₂F produced in Example 1-1-1 while stirring. After further adding 0.08 (1 mmol) of benzene to the resulting homogeneous liquid and stirring, the liquid was transferred to a plastic syringe (starting solution, total volume: 1.75 ml).
Own 100 ml of perfluorohexane were charged into a 180 ml reaction vessel equipped with a 0°C and -78°C two-stage condenser equipped with a gas access port, raw material feed port and an NaF pellet filling pipe and reaction liquid return pipe in between followed by blowing N₂ gas into the liquid for 0. 5 hours at a flow rate of 3 L/Hr. Subsequently, the N₂ gas was replaced with F₂N₂ mixed gas (F₂: 20%, N₂: 80%) and blown in for 0.5 hours at a flow rate of 2.77 L/Hr.
The aforementioned starting solution was supplied to the reaction vessel over the course of 8 hours while maintaining the flow rate of the F₂N₂ mixed gas, after which the gas was blown in for 0.5 hours. The temperature of the reaction liquid was controlled to 18°C to 22°C. Next, 0.56 g (3 mmol) of hexafluorobenzene was dissolved in perfluorohexane and brought to a final volume of 10 ml, and this solution supplies to the reaction vessel over the course of 2 hours while blowing in the F₂N₂ mixed gas at a flow rate of 1 L/Hr, followed by further blowing in the gas for 0.5 hours. Next, the F₂N₂ mixed gas was changed to N₂ gas, and the reactor was purged by blowing the N₂ gas into the liquid for 1 hour at a flow rate of 3 L/Hr. The temperature of the reaction liquid controlled to 21°C to 22°C. GC analysis was carried out on the reaction liquid and CF₃O (CF₂) ₃SO₂F was confirmed to have been formed.
GC-MS mass numbers (relative intensity): 69 (100), 67 (20.5), 119 (8.3), 100 (4.6), 169 (4.1), 50 (1.5), 135 (1.2)

### [b] Production of CF₃O (CF₂) ₃SO₂OCH₂CF₃

Next, 2.76 g (20 mmol) of potassium were added to a reaction vessel at room temperature followed by dropping in 3 g (30 mmol) of CF₃CH₂OH while stirring and then stirring for 4 hours to carry out esterification and obtain CF₃O (CF₂) ₃SO₂OCH₂CF₃. The reaction liquid was filtered using celite as a filtration assistant, washed with water and dried with anhydrous magnesium sulfate. After concentrating, the residue was further vacuum-distilled to fractionate a fraction obtained at 59°C to 61°C at 4.0 kPa and obtain the target compound at a yield of 39%.
¹H-NMR (solvent: CDCl₃, ppm) : 4.72 (q,2H)
¹⁹F-NMR (solvent: CDCl₃, ppm): -124.44 (s,2F) , -110.52 (d,2F) , -85.38 (q,2F), -74.95 (t,3F) , -55.52 (t,3F)

### Example 1-2-2 [Production of CF₃O (CF₂) ₃SO₂F and CF₃O (CF₂) ₃SO₂OCH₂CF₃]

### [a] Production of CF₃O (CF₂) ₃SO₂F

Production was carried out in the same manner as Example 1-2-1 using 0.6 g (30 mmol) of anhydrous hydrofluoric acid, 3.12 g (20 mmol) of the starting compound CH₃O (CH₂) ₃SO₂F and 0.16 g (2 mmol) of benzene (starting solution total volume: 3.2 ml) . However, a -78°C single stage condenser was used for the condenser, the reaction liquid return pipe was omitted from the apparatus of Example 1-2-1, the reaction vessel was changed to that having a volume of 300 ml, and 200 ml of perfluorohexane and 14 g (0133 mmol) of sodium fluoride pellets were charged into the reaction vessel followed by blowing N₂ gas into the liquid for 1 hour at the rate of 3 L/Hr. The N₂ gas was replaced with F₂N₂ mixed gas (F₂: 30%, N₂: 70%) and blown in for 0.5 hours at a flow rate of 3.03 L/Hr.
The aforementioned starting solution was supplied to the reaction vessel over the course of 8 hours while maintaining the flow rate of the F₂N₂ mixed gas, after which the gas was further blown in fop 0. 5 hours. The temperature of the reaction liquid was controlled to 14°C to 16°C. Next, 0.93 g (5 mmol) of hexafluorobenzene was dissolved in perfluorohexane and brought to a final volume of 10 ml, and this solution was supplied to the reaction vessel over the course of 2 hours while blowing in the F₂N₂ mixed gas at a flow rate of 1.13 L/Hr, followed by further blowing in the gas for 0.5 hours. Next, the F₂N₂ mixed gas was changed to N₂ gas, and the reactor was purged by blowing the N₂ gas into the liquid for 1 hour at a flow rate of 3 L/Hr. The temperature of the reaction liquid was controlled to 14°C to 16°C. GC-MS analysis was carried out on the reaction liquid and CF₃O (CF₂) ₃SO₂F was confirmed to have been formed.

### [b] Production of CF₃0(CF₂)₃S0₂0CH₂CF₃

Next, after removing the acidic sodium fluoride (NaHF₂) by filtering the reaction liquid under pressure, 6.9 g (50 mmol) of potassium carbonate were added to a reaction vessel at room temperature followed by dropping in 4 g (40 mmol) of CF₃CH₂OH while stirring and then stirring for 4 hours to carry out esterification. The reaction liquid was filtered using celite as a filtration assistant, washed with water and dried with anhydrous magnesium sulfate. After concentrating, the residue was further vacuum-distilled to obtain the target compound at a yield of 49%.

### [Example 1-2]

Perfluorination of 3-methoxy-1-propanesulfonyl fluoride with fluorine gas and sulfonic acid esterification using trifluoroethanol

CH₃OCH₂CH₂CH₂SO₂F + F₂ → CF₃OCF₂CF₂CF₂SO₂F

CF₃OCF₂CF₂CF₂SO₂F + CF₃CH₂OH → CF₃OCF₂CF₂CF₂SO₂OCH₂CF₃

### Example 2-1 [production of C₂H₅O (CH₂) ₃SO₂F]

Production of the target compound containing an epoxy group was carried out by roughly the same procedure as Example 1-1-2 with the exception of changing the alcohol from methanol to ethanol. Namely, 18.4 g (0.4 mol) of ethanol and 24.4 g (0.2 mol) of 1, 3-propane sultone were charged into the same apparatus as example 1-1-1 and allowed to react for 4 days while refluxing. The reactants were transferred to a recovery flask and concentrated with a rotary evaporator to obtain a viscous liquid. 100 g of chloroform and 0.6 g of N,N-dimethylformamide (DMF) as catalyst were added thereto, a forked connecting tube and a reflux condenser were attached and 47.6 g (0.4 mol) of thionyl chloride were dropped in at room temperature, followed by reacting for 15.5 hours while heating and refluxing in an oil bath. After concentrating the reaction liquid under reduced pressure, a solution containing 100 g of chloroform and 11.6 g (0.2 mol) of potassium fluoride dissolved in 50 g of water was added followed by stirring for 5 days at room temperature. The reaction liquid was separated, the chloroform layer was washed 3 times with water, dried with anhydrous magnesium sulfate concentrate with a rotary evaporator to obtain the target compound by vacuum distillation using a packed column. The amount obtained was 15.42 g, GC purity was 98.5%, yield was 89% and the boiling point was 92°C to 95°C at 2.67 kPa.
¹H-NMR (solvent: CDC1₃, ppm) : 1.19 (t,3H) , 2.18 (m, 2H) , 3.52 (m, 6H) .
¹⁹ F-NMR (solvent_{:} CDCl₃, ppm) : 52.75 (m,lF).

### [Example 2-1]

Synthesis of 3-ethoxy-1-propanesulfonate and 3-ethoxy-1-propanesulfonyl chloride/flouride from 1,3-propane sultone and ethanol

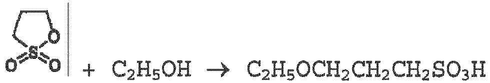

C₂H₅OCH₂CH₂CH₂SO₃H + SOCl₂ → C₂H₅OCH₂CH₂CH₂SO₂Cl

C₂H₅OCH₂CH₂CH₂SO₂Cl + KF → C₂H₅OCH₂CH₂CH₂SO₂F

### Example 2-2 [Production of C2F₅O(CF₂)₃SO₂F and C₂F₅O(CF₂)₃SO₂OCH₂CF₃]

Preparation was carried out in the same manner as Example 1-2-1 using 3.4 g (20 mmol) of the starting compound C₂H₅O(CH₂)₃SO₂F (starting solution total volume: 3.6 ml). Using the same apparatus configuration as example 1-2-2, the same procedure as Example 1-2-2 was carried out with the exception of using 14.62 g (0.35 mol) of powdered sodium fluoride, changing the flow rate of the F₂N₂ mixed gas (F₂: 30%, N₂: 70%) to 3.59 L/Hr, changing the temperature of the reaction liquid to 14°C to 17°C, and changing the temperature of the reaction liquid during introduction of hexafluorobenzene to 12°C to 16°C to obtain the target compound at a yield of 45%. The boiling point was 62°C to 63°C at 2.80 kPa.

C₂F₅O(CF₂)₃SO₂F

GC-MS mass numbers (relative intensity): 119 (100), 69 (59.6), 67 (54.8), 100 (11.9), 31 (10.9), 169 (9.7), 50 (3.2), 147 (2.8)

C₂F₅O(CF₂)₃SO₂OCH₂CF₃

¹H-NMR (solvent: CDCl₃, ppm): 4.72 (q,2H)
¹⁹F-NMR (solvent: CDCl₃, ppm): -124.47 (s,2F), -110.65 (t,2F), -88.79 (t,2F), -87.29 (s,3F), -83.37 (m,2F), -74.97 (q,3F)

### [Example 2-2]

Perfluorination of 3-ethoxy-1-propanesulfonyl fluoride with fluorine gas and sulfonic acid esterification using trifluoroethanol

C₂H₅OCH₂CH₂CH₂SO₂F + F₂ → C₂F₅OCF₂CF₂CF₂SO₂F

C₂F₅OCF₂CF₂CF₂SO₂F + CF₃CH₂OH → C₂F₅OCF₂CF₂CF₂SO₂OCH₂CF₃

### Example 3-1 [Production of n-C₃H₇O(CH₂)₃SO₂F]

The same reaction procedure as Example 1-1-2 was carried out with the exception of using 24g (0.4mol) of n-propyl alcohol for the alcohol, 100 g of chloroform, 0.6 g of N,N-dimethylformamide (DMF) and 47.6 g (0.4 mol) of thionyl chloride and changing the reaction time to 5 Hr, and changing the chloroform to 40 ml of acetonitrile, changing the amount of water to 40 g and changing the reaction time to 3 days. The amount obtained was 15.163 g, GC purity was 99.13%, yield was 84% and the boiling point was 97°C to 98°C at 2.0 kPa.
¹H-NMR (solvent: CDCl₃, ppm): 0.92 (t,3H), 1.15 (m,2H), 2.19 (m,2H), 3.39 (t,2H), 3.53 (m,4H)
¹⁹F-NMR (solvent: CDCl₃, ppm): 52.72 (m,1F)

### [Example 3-1]

Synthesis of 3-propoxy-1-propanesulfonate and 3-propoxy-1-propanesulfonyl chloride/flouride from 1,3-propane sultone and normal propanol

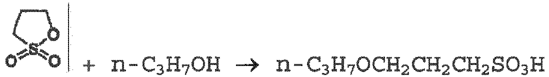

n-C₃H₇OCH₂CH₂CH₂SO₃H + SOCl₂ → n-C₃H₇OCH₂CH₂CH₂SO₂Cl

n-C₃H₇OCH₂CH₂CH₂SO₂Cl + KF → n-C₃H₇OCH₂CH₂CH₂SO₂F

### Example 3-2 [Production of n-C₃F₇O(CF₂)₃SO₂F and n-C₃F₇O(CF₂)₃SO₂OCH₂CF₃]

Preparation was carried out in the same manner as Example 1-2-1 with the exception of changing to 3.8 g (20 mmol) of the starting compound n-C₃H₇O(CH₂)₃SO₂F any 0.93 g (5 mmol) of hexafluorobenzene (starting solution total volume: 4.2 ml). Using the same apparatus configuration as example 1-2-2, the same procedure as Example 1-2-2 was carried out with the exception of using 18 g (0.43 mol) of powdered sodium fluoride, changing the flow rate of the F₂N₂ mixed gas (F₂: 30%, N₂: 70%) to 5.13 L/Hr, changing the temperature of the reaction liquid to 16°C to 17°C, and changing the temperature of the reaction liquid during introduction of hexafluorobenzene to 15°C to 16°C to obtain the target compound at a yield of 47%. The boiling point was 73°C to 75°C at 2.80 kPa.
n-C₃F₇O(CF₂)₃SO₂F₃SO₂F
GC-MS mass numbers (relative intensity): 69 (100), 67 (78.6), 169 (71.8), 100 (17.5), 50 (3.2), 233 (0.5), 235 (0.4)
n-C₃F₇O(CF₂)₃SO₂OCH₂CF₃
¹H-NMR (solvent: CDCl₃, ppm): 4.71 (q,2H)
¹⁹F-NMR (solvent: CDCl₃, ppm): -130.42 (s,2F), -124.42 (d,2F), -110.69 (s,2F), -84.67 (m,2F), -83.24 (m,2F), -81.992 (t,3F), -75.04 (t,3F)

### [Example 3-2]

Perfluorination of 3-propoxy-1-propanesulfonyl fluoride with fluorine gas and sulfonic acid esterification using trifluoroethanol

n-C₃H₇OCH₂CH₂CH₂SO₂F + F₂ → n-C₃F₇OCF₂CF₂CF₂SO₂F

n-C₃F₇OCF₂CF₂CF₂SO₂F + CF₃CH₂OH → n-C₃F₇OCF₂CF₂CF₂SO₂OCH₂CF₃

### Example 4-1 [Production of CH₃O(CH₂)₄SO₂F]

The same reaction procedure as Example 1-1-2 was carried out with the exception of using 33.6 g (1.05 mol) of methanol, 35.6 g (0.26 mol) of 1,4-butane sultone and 5 drops (catalytic amount) of CF₃SO₃H, refluxing for 10 days, using 160 g of chloroform, 1 g of N,N-dimethylformamide (DMF) and 119 g (1 mol) of thionyl chloride, changing the reaction time to 5 Hr, changing the chloroform to 228 ml of acetonitrile, using 30.16 g (0.52 mol) of potassium fluoride, changing the amount of water to 171 g and changing the reaction time to 1 day. The amount obtained was 32.5 g, GC purity was 99.1%, yield was 72% and the boiling point was 104°C to 106°C at 2.53 kPa.
¹H-NMR (solvent: CDCl₃, ppm): 1.75 (m,2H), 2.04 (m,2H), 3.33 (s,3H), 3.45 (m,4H)
¹⁹F-NMR (solvent: CDCl₃, ppm): 52.45 (m,1F)

### [Example 4-1]

Synthesis of 4-methoxy-1-butanesulfonate and 4-methoxy-1-butanesulfonyl chloride/flouride from 1,4-butane sultone and methanol

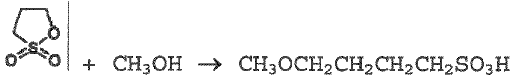

CH₃OCH₂CH₂CH₂CH₂SO₃H + SOCl₂ → CH₃OCH₂CH₂CH₂CH₂SO₂Cl

CH₃OCH₂CH₂CH₂CH₂SO₂Cl + KF → CH₃OCH₂CH₂CH₂CH₂SO₂F

### Example 4-2 [Production of CF₃O(CF₂)₄SO₂F and CF₃O(CF₂)₄SO₂OCH₂CF₃]

Preparation was carried out in the same manner as Example 1-2-1 with the exception of changing to 3.4 g (20 mmol) of the starting compound CH₃O(CH₂)₄SO₂F and changing the benzene to 0.93 g (5 mmol) of hexafluorobenzene (starting solution total volume: 3.9 ml). Using the same apparatus configuration as example 1-2-2, the same procedure as example 1-2-2 was carried out with the exception of using 15.7 g (0.37 mol) of powdered sodium fluoride, changing the flow rate of the F₂N₂ mixed gas (F₂: 30%, N₂: 70%) to 4.39 L/Hr, changing the duration of supplying the starting solution to 6 Hr, changing the temperature of the reaction liquid to 14°C to 18°C, and changing the temperature of the reaction liquid during introduction of hexafluorobenzene to 14°C to 16°C to obtain the target compound at a yield of 40%. The boiling point was 67°C to 69°C at 2.80 kPa.
CF₃O(CF₂)₄SO₂F₃SO₂F₃SO₂F
GC-MS mass numbers (relative intensity): 69 (100), 67 (22.4), 169 (7.7), 100 (5.7), 119 (1.9), 131 (1.6), 135 (1.4)
CF₃O(CF₂)₄SO₂OCH₂CF₃
¹H-NMR (solvent: CDCl₃, ppm): 4.72 (q,2H)
¹⁹F-NMR (solvent: CDCl₃, ppm): -125.70 (m,2F), -120.99 (q,2F), -110.24 (t,2F), -85.56 (q,2F), -74.99 (t,3F), -55.62 (t,3F)

### [Example 4-2]

Perfluorination of 4-methoxy-1-butanesulfonyl fluoride with fluorine gas and sulfonic acid esterification using trifluoroethanol

CH₃OCH₂CH₂CH₂CH₂SO₂F + F₂ → CF₃OCF₂CF₂CF₂CF₂SO₂F

CF₃OCF₂CF₂CF₂CF₂SO₂F + CF₃CH₂OH → CF₃OCF₂CF₂CF₂CF₂SO₂OCH₂CF₃

### Example 5 [Production of C₃F₇O(CF₂)₃SO₂F]

Using an electrolysis bath made of SUS316L and having an effective volume of 480 ml for the electrolysis bath, and using a condenser made of SUS316L for the condenser, the electrolysis bath and condenser were cooled to -21°C with a coolant. Electrodes made of nickel plates having an effective surface area of 0.75 dm²/plate were used for the electrodes, and the electrodes were arranged by mutually separating by a gap of 2 mm.

4.8 g of C₃H₇O(CH₂)₃SO₂F were dissolved in 480 g of anhydrous hydrofluoric acid, and together with a passing a current through electrodes consisting of 8 cathodes and 9 anodes at 9 Ahr, C₃H₇O(CH₂)₃SO₂F was continuously supplied using a pump to carry out electrolytic fluorination. The total amount of raw materials added was 255.47 g, the total amount of current applied was 1209 Ahr, the voltage (when stable) was 5 V to 5.2 V, and the temperature inside the electrolysis bath was 4°C to 6°C.
The perfluorination product was extracted at suitable times from a valve located in the bottom of the electrolysis bath, and a total of 207.9 g were extracted. As a result of analyzing the perfluorination product with a gas chromatograph (capillary column: DB-200), C₃F₇O(CF₂)₃SO₂F was contained at 80.36% as a mixture of the n- and i-forms, and the yield was 29.2%.

The mixture was distilled, a fraction appearing from the top of a distillation column at 109°C to 110°C was collected, and as a result of analyzing the fraction with a gas chromatograph, the n-form accounted for 88.83%, the i-form for 10.06%, and the total amount of C₃F₇O(CF₂)₃SO₂F was 98.89%.
¹⁹F-NMR (solvent: CDCl₃, ppm): -130.08 (s,2F), -124.10 (s,2F), -108.54 (s,2F), -84.33 (m,2F), -82.82 (m,2F), -81.62 (t,3F), 46.32 (m,1F)

### Example 6 [Production of C₄F₉O(CF₂)₃SO₂F]

Electrolytic fluorination was carried out in the same manner as Example 5 with the exception of changing to 6 cathodes and 7 anodes and changing the current flow to 6.75 Ahr from the conditions of Example 5.
The total amount of raw materials added was 272.71 g, the total amount of current applied was 1134 Ahr, the voltage (when stable) was 5.2 V to 5.4 V, and the temperature inside the electrolysis bath was 4°C to 6°C.
The perfluorination product was extracted in the same manner as Example 5, and a total of 138.9 g were extracted. As a result of analyzing the perfluorination product with a gas chromatograph, C₄F₉O(CF₂)₃SO₂F was contained at 81.76% as a mixture of the n- and i-forms, and the yield was 17.6%.

The C₄F₉O(CF₂)₃SO₂F was distilled, a fraction appearing from the top of a distillation column at 130°C to 131°C was collected, and as a result of analyzing the fraction with a gas chromatograph, the n-form accounted for 84.89%, the i-form for 12.94%, and the total amount of C₄F₉O(CF₂)₃SO₂F was 97.83%.
¹⁹F-NMR (solvent: CDCl₃, ppm): -127.11 (s,4F), -126.88 (d,2F), -108.82 (s,2F), -83.58 (m,2F), -82.98 (m,2F), -81.68 (t,3F), 46.03 (m,1F)

### Example 7 [Production of C₃F₇O(CF₂)₃SO₃K and C₄F₉O (CF₂)₃SO₃K]

First, C₃F₇O(CF₂)₃SO₂F was treated for 24 hours at 80°C in a 20% aqueous KOH solution. Next, the reaction liquid was cooled on standing and further cooled with ice water, and after crystals had adequately precipitated, the crystals were filtered out. Moreover, recrystallization was carried out with water, the resulting crystals were adequately dried and then dissolved in acetone, and the filtrate obtained by filtering with a 0.2 µm filter was concentrated and dried with a rotary evaporator followed by vacuum-drying for 24 hours at room temperature.
¹⁹F-NMR (solvent: DMSO-d6, ppm): -129.32 (s,2F), -123.77 (s,2F), -114.59 (s,2F), -83.81 (s,2F), -82.44 (m,2F), -81.55 (t,3F)
Thermal analysis (TG-DTA) result: 397.0°C (decomposition starting temperature)

The same procedure was carried out for the case of C₄F₉O(CF₂)₃SO₂F.
¹⁹F-NMR (solvent: DMSO-d6, ppm): -126.01 (d,4F), -123.79 (s,2F), -111.16 (s,2F), -82.87 (s,2F), -82.44 (m,2F), -80.47 (t,3F)
Thermal analysis (TG-DTA) result: 402.9°C (decomposition starting temperature)

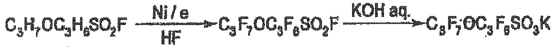

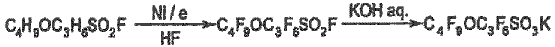

### Example 8 [Measurement of Surface Tension]

The surface tension of C₃F₇O(CF₂)₃SO₃K and C₄F₉O(CF₂)₃SO₃K as well as C₂F₅O(CF₂)₃SO₃K and C₄F₉SO₃K serving as comparative examples was measured in ion exchange water.
Surface tension was measured using for the measuring instrument the Model CBVP-Z Wilhelmy Automated Surface Tensiometer (Kyowa Interface Science Co., Ltd.), and the measuring temperature was 23°C. The results are shown in Table 1.

**[Table 1]**

| Concentration in water (ppm) | 100 | 500 | 1000 | 2500 | 5000 | |
|---|---|---|---|---|---|---|
| C₃F₇OC₃F₆SO₃K | 69.8 | 62.9 | 58.1 | 47.4 | 37.4 | Surface tension (mN/m) |
| C₄F₉OC₃F₆SO₃K | 66.3 | 54.8 | 46.9 | 34.4 | 23.6 | |
| C₂F₅OC₃F₆SO₃K | 71.5 | 67.8 | 64.5 | 57.4 | 48.8 | |
| C₄F₉SO₃K | 72.2 | 71.5 | 70.0 | 67.4 | 64.1 | |

As shown in Table 1, C₃F₇O (CF₂) ₃SO₃K and C₄F₉O(CF₂)₃SO₃K were clearly determined to demonstrate a high ability to lower surface tension in comparison with C₂F₅O(CF₂)₃SO₃K and C₄F₉SO₃K.

### INDUSTRIAL APPLICABILITY

According to the present invention, molecular design can be carried out with comparative inexpensive hydrocarbon compounds, and perfluorinated compounds can be obtained while retaining the structure thereof. In addition, in addition to the low cost, yield is favorable. Consequently, the present invention in highly useful as a method for synthesizing various novel compounds by using alternative compounds to conventional perfluoroalkylsulfonic acids and derivatives thereof.

## Claims

1. A method for producing a fluorine-containing ether sulfonic acid compound, comprising: producing a perfluorosulfonic acid having an ether structure and a derivative R_{F}¹OR_{F}²SO₂X thereof (wherein, R_{F}¹ and R_{F}² represent groups in which hydrogen atoms in the R_{H}¹ and R_{H}² groups have been substituted with fluorine atoms and X represents -OH, an alkoxy group or a halogen) by perfluorinating a sulfonyl halide represented by the general formula R_{H}²OR_{H}¹SO₂Y (wherein, R_{H}¹ and R_{H}² respectively represent a hydrocarbon group having 1 to 4 carbon atoms and Y represents fluorine or chlorine).

2. The method for producing a fluorine-containing ether sulfonic acid compound according to claim 1, wherein the sulfonyl halide is such that :
the R_{H}¹ is a hydrocarbon group (linear or branched) having 3 carbon atoms the case the R_{H}²is a hydrocarbon group having 1 carbon atom,
the R_{H}¹ is a hydrocarbon group having 1 carbon atom, hydrocarbon group (linear or branched) having 3 atoms or hydrocarbon group (linear or branched) having 4 carbon atoms in the case the R_{H}² is a hydrocarbon group (linear or branched) having 3 carbon atoms, and
the R_{H}¹ is a hydrocarbon group having 1 carbon atom or a hydrocarbon group (linear or branched) having 3 carbon atoms in the case the R_{H}² is a hydrocarbon group (linear or branched) having 4 carbon atoms.

3. The method for producing a fluorine-containing ether sulfonic acid compound according to claim 1 or 2, wherein the sulfonyl halide is sulfonyl fluoride (R_{H}²OR_{H}¹SO₂F) , the sulfonyl fluoride is added to hydrofluoric acid to obtain a solution containing a hydrogen bonded complex, and this is supplied to a reaction solvent together with F₂ gas and then perfluorinated in a liquid phase to produce perfluorosulfonic acid having an ether structure and a derivative R_{F}¹OR_{F}²SO₂X thereof (wherein, R_{F}¹and R_{F}² represent groups in which hydrogen atoms in the R_{H}¹ and R_{H}² groups have been substituted with fluorine atoms and X represents -OH, an alkoxy group or a halogen).

4. The method for producing a fluorine-containing ether sulfonic acid compound according to claim 1 or 2, wherein the sulfonyl halide is sulfonyl chloride (R_{H}²OR_{H}¹SO₂Cl) , and the sulfonyl chloride is added to hydrofluoric acid to convert to sulfonyl fluoride and obtain a solution containing a hydrogen bonded complex, and this is supplied to a reaction solvent together with F₂ gas and then perfluorinated in a liquid phase to produce a perfluorosulfonic acid having an ether structure and a derivative R_{F}¹OR_{F}²SO₂X thereof (wherein, R_{F}¹ and R_{F}² represent groups in which hydrogen atoms in the R_{H}¹ and R_{H}² groups have been substituted with fluorine atoms and X represents -OH, an alkoxy group or a halogen).

5. The method for producing a fluorine-containing ether sulfonic acid compound according to claim 1 or 2, wherein the sulfonyl halide is sulfonyl fluoride (R_{H}²OR_{H}¹SO₂F) , and the perfluorination is carried out by electrolytically fluorinating the sulfonyl fluoride in anhydrous hydrofluoric acid.

6. A production method of the perfluorosulfonic acid having an ether structure and a derivative R_{F}¹OR²SO₂X thereof according to claim 3 or 4, comprising: carrying out the reaction by preliminarily adding and suspending NaF or KF in the liquid phase fluorination reaction liquid as an adsorbent of hydrofluoric acid.

7. A production method of the perfluorosulfonic acid having an ether structure and a derivative R_{F}¹OR_{F}²SO₂X thereof according to any of claims 1 to 6, comprising: converting a fluorination reaction product (R_{F}²OR_{F}¹SO₂F) in the reaction liquid to a sulfonic acid ester (R_{F}²OR_{F}¹SO₂OR³) using a base and an alcohol R³OH, and then separating purifying by distillation.

8. A method for producing a hydrocarbon sulfonyl fluoride having an ether structure (alkoxyalkylsulfonyl fluoride), comprising: a step wherein an alkoxide, obtained by reacting CH₃OM, C₂H₅OM or a linear or branched alcohol having 3 to 4 carbon atoms with a metal M, M-H or CH₃OM (wherein, M represents Na, K or Li) , is reacted with X¹-R_{H}¹-SO₂-X² (wherein, X¹ represents Cl or Br, R_{H}¹ represents a linear alkyl group having 1 to 4 carbon atoms, and X² represents ONa, OK, Cl or Br) to synthesize R²-O-R_{H}¹-SO₂-X² (wherein,R²-O- represents an alkoxy group equivalent to the alkoxide), followed by subjecting to the action of a chlorinating agent to obtain R_{H}²-O-R_{H}¹-SO₂-Cl, and further converting to R_{H}²-O-R_{H}¹-SO₂-F in an aqueous solution containing KF.

9. A method for producing a hydrocarbon sulfonyl fluoride having an ether structure (alkoxyalkylsulfonyl fluoride), comprising: a step wherein CH₃OH, C₂H₅OH or a linear or branched alcohol having 3 to 4 carbon atoms as reacted directly with 1, 3-propane sultone or 1, 4-butane sultone to synthesize R²-O-R_{H}¹-SO₂-OH (wherein, R²-O- represents an alkoxy group equivalent to the alkoxide, and R_{H}¹ represents a linear alkylene derived from the sultone), followed by subjecting to the action of a chlorinating agent to obtain RH²-O-R_{H}¹-SO₂-Cl, and further converting to R_{H}²-O-R_{H}¹-SO₂-F in a KF- organic solvent-water system.

10. A method for producing a hydrocarbon sulfonyl fluoride having an ether structure (alkoxyalkylsulfonyl fluoride), comprising: a step wherein an alkoxide, obtained by reacting CH₃OM, C₂H₅OM or a linear or branched alcohol having 3 to 4 carbon atoms with a metal M, M-H or CH₃OM (wherein, M represents Na, K or Li), is reacted directly with 1,3-propane sultone or 1,4-butane sultone to synthesize R²-O-R_{H}¹-SO₂-OM (wherein, R²-O-represents an alkoxy group equivalent to the alkoxide, and R_{H}¹ represents a linear alkylene derived from the sultone), followed by subjecting to the action of a chlorinating agent to obtain R_{H}²-O-R_{H}¹-SO²-Cl, and further converting to R_{H}²-O-R_{H}¹-SO₂-F in an aqueous solution containing KF.

11. A fluorine-containing ether sulfonic acid compound that is a compound represented by general formula R_{F}¹OR_{F}²SO₂X (wherein, RF¹ and R_{F}² respectively represent a perfluoroalkyl group having 1 to 4 carbon atoms, and X represents -OH, an alkoxy or a halogen) , wherein
the R_{F}¹ is a perfluoroalkyl group (linear or branched) having 3 carbon atoms in the case the R_{F}² is a perfluoroalkyl group having 1 carbon atom,
the R_{F}¹ is a perfluoroalkyl group having 1 carbon atom, a perfluoroalkyl group (linear or branched) having 3 carbon atoms or a perfluoroalkyl group (linear or branched) having 4 carbon atoms in the case the R_{F}² is a perfluoroalkyl group (linear) having 3 carbon atoms, and
the R_{F}¹ is a perfluoroalkyl group having 1 carbon atom or a perfluoroalkyl group (linear or branched) having 3 carbon atoms in the case the R_{F}² is a perfluoroalkyl group (linear) having 4 carbon atoms.

12. A surfactant containing a compound represented by general formula R_{F}¹OR_{F}²SO₃M (wherein, R_{F}¹ and R_{F}²respectively represent a perfluoroalkyl group having 1 to 4 carbon atoms, and M represents Li, Na, K or NH₄) , wherein
the R_{F}¹ is a perfluoroalkyl group (linear or branched) having 3 carbon atoms in the case the R_{F}² is a perfluoroalkyl group having 1 carbon atom,
the R_{F}¹ is a perfluoroalkyl having 1 carbon atom, a perfluoroalkyl group (linear or branched) having 3 carbon atoms or a perfluoroalkyl group (linear or branched) having 4 carbon atoms in the case the R_{F}² is a perfluoroalkyl group (linear) having 3 carbon atoms, and
the R_{F}¹ is a perfluoroalkyl group having 1 carbon atom or a perfluoroalkyl group (linear or branched) having 3 carbon atoms in the case the R_{F}² is a perfluoroalkyl group (linear) having 4 carbon atoms.
